# EUROPEAN PATENT APPLICATION

(11) **EP 2 198 818 A1**
(43) Date of publication of application: **23.06.2010**
(21) Application number: 08021907.4
(22) Date of filing: 17.12.2008
(51) Int. Cl.: A61F 13/56

(54) **Undergament with marking**

(71) Applicant: Attends Healthcare AB, 578 24 Aneby (SE)
(72) Inventor: Svensson, Ida, 55450 Jönköping (SE); Hakansson, Mikael, 573 37 Aneby (SE); Persson, Christer, 578 31 Aneby (SE); Lindqvist, Arne, 152 57 Södertälje (SE)
(74) Representative: Jacobsson, Peter

(57) **Abstract**

The invention deals with an undergarment for use with an absorbent article provided with attachment means for attaching the absorbent article to the undergarment, wherein the undergarment is provided with at least one marking for facilitating placement of the article in the undergarment, wherein the at least one marking corresponds to the placement of at least a portion of the attachment means of the article in the undergarment.

## Description

### Technical field

The present document relates to an undergarment provided with markings for use with an absorbent article. The document particularly relates to an undergarment for use with an incontinence pad.

### Background

When using absorbent articles, such as incontinence or menstrual protection articles, the article is usually placed either in the undergarment that the user normally uses or in a specially designed undergarment for use with the specific article. The absorbent article may be e.g. sanitary or incontinence napkins, towels or pads.

The article is usually provided with some sort of adhesive in order to be fastened in the crotch region of the undergarment. To achieve an optimal protection against leakage it is important that the article is placed correctly in the undergarment.

Further it is often desirable for the users of e.g. incontinence articles to have a discrete protection, i.e. not notable from the outside of the undergarment.

WO 97/22321 discloses a hygienic panty and a quick-attach pad. The panty is provided with a thin yet absorbent crotch panel for holding excess fluids not absorbed by the pad including a fluid absorbent layer overlying a fluid barrier. The upper surface of the layer includes yam loops for being releasably held by hook fasteners of the pad. The crotch panel may be stitched onto the panty and include a target marking approximately corresponding to the peripheral shape of the pad, thereby marking the placement of the pad on the crotch panel.

Such target marking for placing an absorbent pad is not discrete to a user. i.e. a panty equipped with such a target marking clearly shows that the user has some kind of incontinence problem. Further since different pads are differently large, a panty has to be equipped with different target markings depending on the kind of pad, e.g. size of the pad, what type of incontinence problem the user has etc.

There is hence a need for an undergarment that indicates correct placement of many types and sizes of incontinence products, which makes it easy for the user to correctly place the article in the undergarment, and/or which is discrete for the user.

### Summary

It is an object of the present disclosure, to provide an improved or alternative undergarment, which eliminates or alleviates at least some of the disadvantages of the prior art undergarments.

The object is wholly or partially achieved by an undergarment according to the appended independent claims. Embodiments are set forth in the appended dependent claims, and in the following description and drawings.

According to a first aspect there is provided an undergarment for use with an absorbent article provided with attachment means for attaching the absorbent article to the undergarment, wherein the undergarment is provided with at least one marking for facilitating placement of the article in the undergarment, wherein the at least one marking corresponds to, or indicates, the placement of at least a portion of the attachment means of the article on the undergarment.

Persons using absorbent articles have different incontinence problems. For example, a person may have a leakage of urine or a leakage of excrements. Further, a person may have light or more severe incontinence problems, meaning that persons may be in need of articles with more or less absorbing capacity. To be optimal for the user, such articles would have different outer shapes depending on the purpose of the article. Thereby, for a panty as described in WO 97/22321, an undergarment of a certain size, e.g. medium (M) would need to have different markings depending on the outer shape of the absorbent article, i.e. different markings for each different absorbent article. A lot of different markings in an undergarment makes correct placement difficult for the user. Alternatively, it may be necessary to produce different undergarments for one size (e.g. M) depending on the type of article to be placed in the undergarment. This results in many different types of undergarments, which makes production of the undergarments more costly. Also, it will be difficult for a person to take the correct undergarment to the incontinence article to be used.

The fact that, according to the invention, the marking in the undergarment is arranged to specifically indicate the placement of the attachment means of the absorbent article instead of indicating the peripheral shape of the absorbent article could greatly simplify the correct placement of the article. For example, by producing different types of absorbent articles for which the attachment means are arranged in the absorbent articles such that for optimal use, the attachment means will be placed in the undergarment at more or less the same place irrespective of the type of article, an undergarment could be produced that indicates correct placement for many types of incontinence articles with only one type of marking on the undergarment. Furthermore, by having a marking on the undergarment corresponding to the placement of an attachment means of the absorbent article, as in the present invention, it is possible to produce only one or at least a small number of different underwear of a certain size each indicating correct placement of an absorbent article irrespective of size and type of the article.

Further, that the marking only corresponds to the shape of the attachment means or a portion of the attachment means, and e.g. not to the shape of the entire article may further make the marking and therefore also the undergarment more discrete and hence not revealing that the user is in need of an absorbent article.

Furthermore, since the marking indicates the placement of an attachment means of the absorbent article, a user will get a clear guidance to an optimal placement of the article.

According to an embodiment, the undergarment is provided with at least two separate markings, each marking indicating correct placement of an attachment means of an absorbent article. Thereby, a user will get an even clearer guidance to an optimal placement of the absorbent article compared to if only one marking is used.

The absorbent article may be selected from the group consisting of incontinence and menstrual protection articles. The article may be an incontinence pad.

According to an embodiment, the marking may comprise loop fasteners for interaction with corresponding hook fasteners arranged on the absorbent article. By the attachment means comprising hook fasteners and the markings loop fasteners, there may be provided an undergarment that is easily reusable in that the undergarment may be washed repeatedly without loosing the effect of the loop fastener marking.

According to another embodiment, the marking may be embossed into the undergarment, e.g. sewn.

According to the first aspect the marking may correspond to the shape and size of adhesive strips or bands arranged on the absorbent article.

The marking further may be arranged to correspond to the placement of the absorbent article according to male or female anatomy respectively. To this end, arranging the markings such as to correspond to the different need of placement depending on whether the user is a woman or a man, a more safe and correct placement of the article may be achieved.

The marking may correspond to the peripheral shape of the attachment means. That means that the marking essentially shows the outline of the attachment means. Thereby, placing of an article in the undergarment is facilitated.

The marking may correspond to the entire area of the attachment means. This means that the entire area, such as an adhesive strip or a strip of hook or loop fasteners, or pluralities of such strips may be outlined as a marking on the undergarment. For example, the marking may comprise a coloured or patterned area. The marking may also be a material of a different type than the material of the undergarment.

The marking may comprise at least two separate portions placed at a distance from each other, and the separate portions of the marking may be placed at a front and a rear part of the crotch region of the undergarment. Further, the portions may correspond to a peripheral shape of the attachment means.

This means that the marking may, e.g. correspond to the outline of a front and rear portion of the attachment means, and hence be placed separately at the front and rear part of the undergarment. The marking may alternatively correspond to the outline of the side portions of the attachment means and hence be placed at portions of the crotch region displaced from a longitudinal axis of the undergarment. This may facilitate a simplified placement of the absorbent article in the undergarment, yet still be discrete.

According to another aspect of the invention, there is provided a kit of parts comprising an undergarment according to the first aspect and at least one absorbent article provided with attachment means for attaching the absorbent article to the undergarment. By such a kit, a user will at the same time get hold of an undergarment of her/his size together with a number of absorbent articles adapted for his/her special need, articles which at the same time will fit the undergarment. By adapting the placement of the attachment means in the absorbent articles to the markings of the undergarment, or vice versa, the user can easily place the article in the undergarment when needed. When the article has been placed in the undergarment, the undergarment will function as a pull-on diaper. l.e. it can be taken on and off many times and the fixation of the article in the undergarment will still be stable. Also, the undergarment can be taken off the body of the user; a used article can be disposed with, and replaced by another article of the kit of parts in the undergarment.

### Brief Description of the Drawings

Embodiments of the present solution will now be described, by way of example, with reference to the accompanying schematic drawings.

Fig 1a is a schematic perspective view of an undergarment with markings and an absorbent article to be placed in the undergarment.

Fig 1b is a schematic perspective view of the undergarment of fig. 1a and a different absorbent article to be placed in the undergarment.

Fig. 2 is a schematic perspective view of another absorbent article with markings and the absorbent article of fig. 1a.

Fig. 3 is a schematic perspective view of another undergarment with markings and an absorbent article to be placed in the undergarment.

### Description of Embodiments

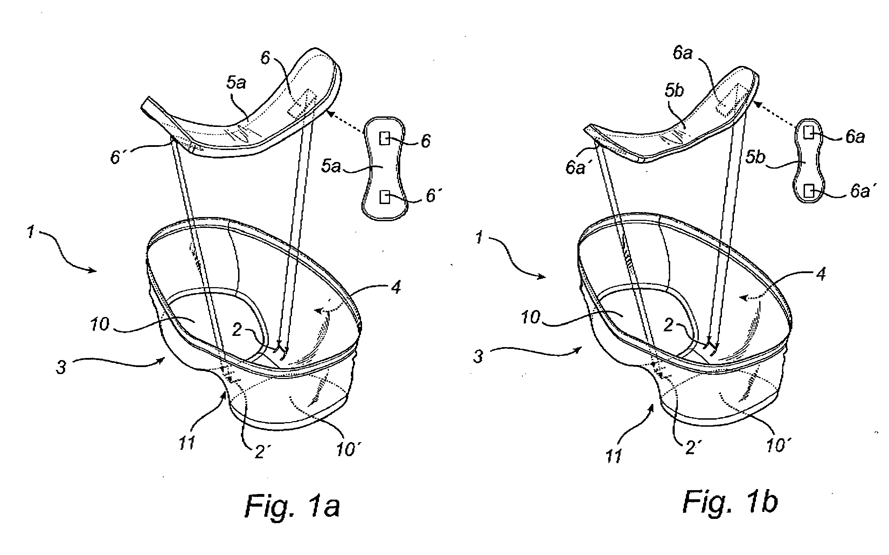

Figs 1 a and 1b illustrate an undergarment 1 comprising a front part 3, a rear part 4, two openings 10, 10' for the legs of a user of the undergarment, and a crotch region 11, connecting the leg openings 10, 10' and the front 3 and rear 4 parts. The inside of the front part 3 is intended to face the front side of a person's body. The crotch region 11 of the undergarment may be designed such as to provide optimal comfort to the wearer, while still comprising a sufficient area to place the absorbent article on. Even though an undergarment of a boxer-shorts-type is illustrated in the figures of the present disclosure, it shall be readily understood that the undergarment may have any type of design, such as briefs and hipsters.

According to fig. 1a, the undergarment 1 is further provided with two markings 2, 2', each shaped as a "C", arranged in the crotch region 11 of the undergarment 1, on the inside of the undergarment, i.e. the side of the undergarment that faces the body of a user, when the undergarment is worn by a user. The first marking 2 is placed closer to the rear part of the undergarment than the second marking 2'. The markings 2, 2' indicate correct placement of an absorbent article in the undergarment.

Figure 1 a further illustrates an exemplary absorbent article in shape of a pad 5a that may be placed in the undergarment 1. The pad comprises two attachment means 6, 6', arranged on one side of the pad, the side intended to face the undergarment, when the pad is placed in the undergarment. The attachment means 6, 6' may be e.g. of hook-and-loop type or an area with adhesive, preferably covered by a cover when not used, or similar. A first attachment means 6 is placed at a rear portion of the pad and a second attachment means 6' is placed at a front portion of the pad, in a longitudinal direction of the pad. The front portion of the pad is intended to be placed at the front part 3 of the undergarment and the rear portion of the pad is intended to be placed at the rear part 4 of the undergarment.

The markings 2, 2 of the undergarment 1 indicate correct placement of the attachment means 6, 6' arranged on the pad. When a user is about to place the pad 5a inside the undergarment 1, the user places the pad such that the borders of each attachment means 6, 6' is in line with its corresponding C-marking 2, 2', as illustrated by the arrows of figure 1a. l.e. the markings indicate the peripheral shape of the ends of each attachment means. Further, the markings indicate the peripheral shape of the attachment means ends that are furthest away from each other, to facilitate placement of the pad. The pad 5a has two separate attachment means 6, 6', although, the undergarment 1 of fig. 1 a will also function for a pad having only one attachment means extending over a larger area from the front portion to the rear portion of the pad. In that case, the markings 2, 2' of the undergarment 1 will indicate placement of opposite ends of such a larger attachment means.

The markings 2, 2' may have any shape or size or be placed essentially anywhere on the undergarment, as long as the markings match a correct placement of an attachment means of a pad to be placed in the undergarment.

The markings of fig. 1a are embossed or printed on the undergarment.
Fig. 1b shows the same undergarment as in fig. 1a with the same marking. Fig. 1b also illustrates an absorbent pad 5b with a smaller size than the absorbent pad 5a of fig. 1a. As seen from the figure, especially of the top views of the pad, the attachment means 6a, 6a' are placed closer to the longitudinal ends of the pad, compared to the attachment means 6, 6' of the pad 5a shown in figure 1a. Consequently, the markings 2, 2' of the underwear indicate correct placement of the ends of the attachment means for both the large absorbent pad 5a illustrated in figure 1a and for the small absorbent pad 5b illustrated in figure 1b.

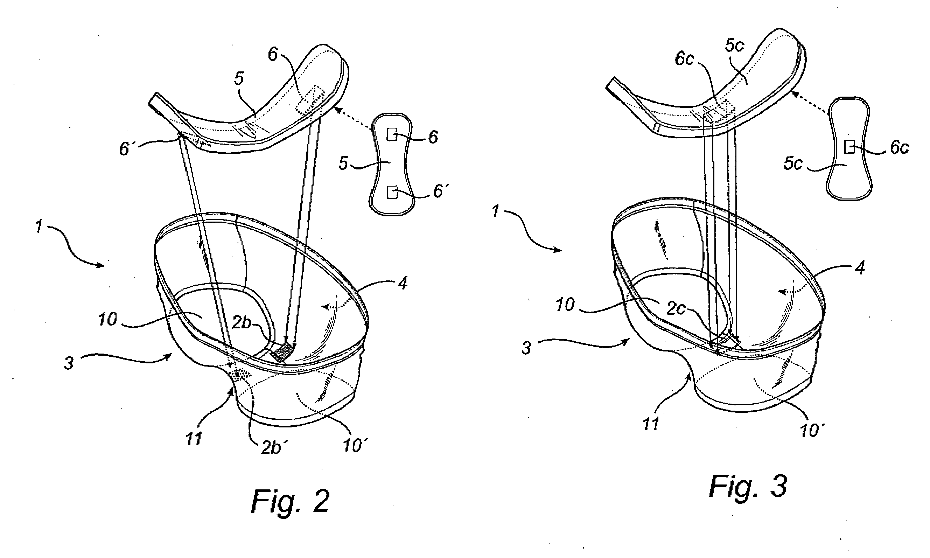

Fig. 2 illustrates another undergarment 1. The undergarment of fig. 2 differs from the undergarment of fig. 1a and 1b by a different shape of the markings 2b, 2b'. In this figure the markings indicate the entire shape of the attachment means of the absorbent article, thereby corresponding to the correct placement of the attachment means 6, 6' of an absorbent article such as the absorbent article 5 in the undergarment 1. The entire shape of the attachment means could be indicated by marking the entire contour of the attachment means or by marking the entire area for the attachment means, e.g. by a coloured area.
Figure 3 shows an alternative undergarment where the marking 2c corresponds to correct placement of a sole attachment means 6c of another pad 5c. The marking 2c indicates the entire shape of the attachment means 6c.

The markings may alternatively (not shown) be placed at side parts of the crotch region.

According to one alternative embodiment the markings 2, 2' comprise loop fasteners arranged to engage hook fasteners on the article. The hooks on the absorbent pad may be arranged as a separate hook part that is detachably arranged to the pad. Thereby, the hook part may be attached and detached from the pad, such that the hook part can be reused on a new pad when the original pad has been used and is to be disposed with. Such hook and loop fasteners may be of a Velcro®-type. In an alternative, the markings may comprise hook fasteners arranged to engage loop fasteners on the article. Other kind of fixation means may also be used to attach a pad to the undergarment.

According to yet an alternative the marking may be arranged to correspond to at least a portion of an adhesive strip or band arranged on the article.

The marking could e.g. have one colour or pattern for a male user and another colour or pattern for a female user.

The marking is advantageously such that it may withstand repeated washing of the undergarment, and the wear from repeated changing of the article. The marking is further preferably such that it is discrete, i.e. not visible from the outside of the undergarment.

## Claims

1. An undergarment (1) for use with an absorbent article (5) provided with attachment means (6, 6') for attaching the absorbent article to the undergarment, wherein the undergarment (1) is provided with at least one marking (2, 2') for facilitating placement of the article in the undergarment, **characterized in that** the at least one marking (2, 2') corresponds to the placement of at least a portion of the attachment means of the article in the undergarment.

2. The undergarment (1) as claimed in claim 1, wherein the at least one marking (2, 2') comprises at least two separate markings.

3. The undergarment (1) as claimed in claim 2, wherein a first of the at least two separate markings corresponds to placement of a first end of the attachment means of the article in the undergarment and a second of the at least two separate markings correspond to placement of a second end of the attachment means.

4. The undergarment (1) as claimed in any one of claims 1-3, wherein the at least one marking (2, 2') comprises loop fasteners for interaction with corresponding hook fasteners arranged on the absorbent article (5).

5. The undergarment (1) as claimed in any one of claims 1-4, wherein the at least one marking (2) corresponds to adhesive strips or bands arranged on the absorbent article (5).

6. The undergarment (1) as claimed in any one of the preceding claims, wherein the at least one marking (2) corresponds to at least parts of the peripheral shape of the attachment means (6).

7. The undergarment (1) as claimed in any one of claims 1-5, wherein the at least one marking (2, 2') corresponds to the entire area of an attachment means (6).

8. The undergarment (1) as claimed in claim 7, wherein the at least one marking (2, 2') comprises a coloured or patterned area.

9. The undergarment (1) as claimed in any one of the preceding claims, wherein the at least one marking (2, 2') comprises at least two separate portions placed at a distance from each other, each portion corresponding to placement of an end of the attachment means of the absorbent article.

10. The undergarment (1) as claimed in claim 9, wherein the at least two separate portions of the at least one marking (2, 2') are placed one at a front part (3) of the undergarment and the other at a rear (4) part of the undergarment.

11. The undergarment (1) as claimed in any one of claims 9 or 10, wherein the portions of the at least one marking corresponds to a peripheral shape of the attachment means (6).

12. A kit of parts comprising an undergarment according to any of claims 1-11 and at least one absorbent article (5a; 5b; 5c) provided with attachment means (6, 6') for attaching the absorbent article to the undergarment.
